# EUROPEAN PATENT APPLICATION

(11) **EP 2 626 084 A1**
(43) Date of publication of application: **14.08.2013**
(21) Application number: 13154593.1
(22) Date of filing: 08.02.2013
(51) Int. Cl.: A61K 49/00

(54) **Combination of sodium chloride and epithelial sodium channel blocker**

(30) Priority: 10.02.2012 EP 12154853
(71) Applicant: Westfälische Wilhelms-Universität Münster, 48149 Münster (DE)
(72) Inventor: Oberleithner, Hans, 48149 Münster (DE)
(74) Representative: Schiweck, Weinzierl & Koch

(57) **Abstract**

Provided is a combination of an epithelial sodium channel blocker and sodium chloride. The combination comprises sodium chloride in an amount of 1.5 g or more. Provided is also a method of diagnosing an increased risk of developing hypertension and/or of stratifying the risk of developing hypertension. A further method is a method of determining whether the blood pressure of a subject is sensitive to sodium intake. These methods comprise administering an epithelial sodium channel blocker and sodium chloride to the subject. After waiting for about 10 minutes or more, at least one of systolic and diastolic blood pressure of the subject is determined, and the blood pressure is compared to a control measurement or to a predetermined reference value. A temporary increase in systolic and/or diastolic blood pressure compared to the control measurement indicates a risk of a disorder, or that the blood pressure of the subject is sensitive to sodium intake. Provided is also a kit with a first container that comprises sodium chloride, but is at least essentially void of an epithelial sodium channel blocker. A second container of the kit comprises a combination of an epithelial sodium channel blocker and sodium chloride.

## Description

### FIELD OF THE INVENTION

The present invention relates to a combination of an epithelial sodium channel blocker and sodium chloride as well as uses of such combination. Provided is also a method of diagnosing an increased risk of developing arterial hypertension and/or of stratifying the risk of developing arterial hypertension, as well as a method of determining whether the blood pressure of a subject is sensitive to sodium intake.

### BACKGROUND OF THE INVENTION

Circulatory disorders are worldwide the number one cause of death. Their main cause is the wear-out of arterial blood vessels over the course of life. Late effects of impaired vessels are stroke (apoplexy) and cardiac infarction. Hypertension (arterial hypertension) is regarded the most important factor in the pathogenesis of cardiovascular disorders (Meneton, P, et al., Physiol Rev (2005) 85, 679-715). With more than eight billion Euro per year alone in Germany, as well as the resulting follow-up costs the treatment of hypertension is the most expensive of all diseases. Almost half of the worldwide population above 50 years of age suffers from hypertension and will, with high likelihood, suffer from the long-term effects. The main problem in this regard is early diagnosis.

Hypertension is diagnosed when systolic blood pressure values are consistently above 140 mm Hg and/or diastolic blood pressure values are above 90 mm Hg. It is recommended that individuals with values in the range of 120-139/80-89 be categorized as having prehypertension (Sutters, M, in: Current Medical Diagnosis and Treatment (2013) McGraw Hill, Ch 11). Prehypertension often develops into hypertension, so that even low risk prehypertensive patients should be monitored annually (ibid.).

Complications associated with manifest hypertension are irreversible alterations of the vasculature and the heart as well as atherosclerosis connected to long lasting hypertension. Chronic hypertension further leads to nephrosclerosis. At the stage of irreversible alterations only "damage control" (by lifelong application of drugs, e.g., antihypertensives), can be done, but no healing as such is achievable. The onset of essential hypertension is generally at an age from 25 to 55 years. Only for about 5 % of patients with hypertension a specific cause can be identified such as a drug-induced effect or a chronic kidney disease. Unfortunately only repeated measurements of the arterial blood pressure reveals existing (manifest) hypertension, so that "arterial hypertension" is only diagnosed at a stage where the blood vessels in all likelihood are already irreversibly damaged (proliferation of the connective tissue, rarefication of elastic fibers of the main arteries, hypertrophy of vascular smooth muscle).

It has been suggested that an increased salt intake could be related to extracellular fluid expansion and a rise in blood pressure. In this regard the renin-angiotensin system has been suggested as mediating adverse cardiac and renal effects of excessive salt intake. It has also been speculated that increased salt levels in cerebrospinal fluid, as a result of excess dietary salt, activate sodium sensing circumventricular organs of the brain, thereby increasing sympathetic nerve activity, which leads to vasoconstriction. Chronic excessive sodium intake has also been shown to raise secretion of endogenous ouabain by the adrenal cortex, a glycoside hormone that inhibits the Na/K ATPase (Blaustein, MP, et al., Am J. Physiol Regul Integr Comp Physiol (2006) 290, R514-R523).

These indications point to certain mechanistic aspects of elevated intake of sodium chloride. While a genetic predisposition has been proposed, there is, however, so far no clear indication on a connection between sodium absorption and increased blood pressure. Let alone is there a test that would be suitable for predicting whether a subject is at an increased risk of developing hypertension.

Nevertheless the search for means of early diagnosis of a potential hypertension, i.e. of identifying an increased risk of developing hypertension, is of central interest, inter alia due to its tremendous economic importance. Unfortunately hopes that it might be possible to predict development of hypertension via a "genetic fingerprint" have not been fulfilled, as numerous studies on the human genome over the last two decades have shown (Lupton SJ, et al., Twin Res Hum Genet (2011) 14, 295-304). Hypertension cannot be ascribed to a few dysfunctional genes or their gene products.

It is therefore an object of the present invention to provide means that allow the diagnosis of an increased risk of developing hypertension.

### SUMMARY OF THE INVENTION

The present invention provides a combination that can be used for a quick and simple test in medical diagnosis as well as in determining whether the blood pressure of a subject is sensitive to sodium intake. Typically such a test can thereby be used to indicate whether the vascular system of an individual is sensitive to sodium intake. The test can be used to assess at a pre-hypertension state whether a subject will be in need of hypertension therapy and can be used in prognosis and risk assessment. Hence, precautionary measures in terms of medical prevention can be taken already at a stage where no damage has occurred in the subject's organs and circulatory system.

In a first aspect the present invention provides a combination of an epithelial sodium channel blocker and sodium chloride. The combination contains sodium chloride in an amount of 1.5 g or more.

In some embodiments the combination consists of sodium chloride and an epithelial sodium channel blocker. In some embodiments the combination essentially consists of sodium chloride and an epithelial sodium channel blocker. In some embodiments the combination further includes a monosaccharide such as for instance glucose. In some embodiments the combination includes a disaccharide. In some embodiments the combination consists of sodium chloride, an epithelial sodium channel blocker and a monosaccharide. In some embodiments the combination consists of sodium chloride, an epithelial sodium channel blocker and a disaccharide. In some embodiments the combination essentially consists of sodium chloride, an epithelial sodium channel blocker and a monosaccharide. In some embodiments the combination essentially consists of sodium chloride, an epithelial sodium channel blocker and a disaccharide.

In some embodiments the combination is for use in diagnosis and/or in stratifying the risk of a disorder. In some embodiments the combination is for use in diagnosis of an increased risk of developing hypertension and/or stratifying the risk of developing hypertension.

The diagnosis and/or stratifying the risk of a disorder includes in some embodiments administration of an epithelial sodium channel blocker and sodium chloride to the subject. The diagnosis and/or stratifying the risk of a disorder may further include waiting for about 10 minutes or more. Further, the diagnosis and/or stratifying the risk of a disorder may include a determination of at least one of systolic and diastolic blood pressure of the subject. The diagnosis and/or stratifying the risk of a disorder may also include a comparison of the blood pressure to a control measurement or to a predetermined reference value. An increase in systolic and/or diastolic blood pressure compared to the control measurement indicates a risk of a disorder.

In this regard the present invention in a second aspect relates to a method of diagnosing an increased risk of developing hypertension and/or stratifying the risk of developing hypertension. The method includes administering an epithelial sodium channel blocker and sodium chloride to the subject. The method also includes waiting for about 10 minutes or more. Further, the method includes determining at least one of systolic and diastolic blood pressure of the subject. The method also includes comparing the blood pressure to a control measurement or to a predetermined reference value. An increase in systolic and/or diastolic blood pressure compared to the control measurement indicates a risk of a disorder.

In some embodiments repeated sessions of the method are carried out, such as periodic session. In one embodiment one session is carried out at least essentially without administering an epithelial sodium channel blocker, for example by administering only NaCl or a composition that includes NaCl but no epithelial sodium channel blocker. A further session is carried out with a combination of NaCl and an epithelial sodium channel blocker. Blood pressure values after administration of the combination of NaCl and the epithelial sodium channel blocker can then be compared to blood pressure values after administration of NaCl/composition without the epithelial sodium channel blocker. A significant increase in blood pressure values after administration of the combination, when compared to blood pressure values after administration of NaCl/composition but no epithelial sodium channel blocker, indicates a risk of a disorder.

In a third aspect the present invention provides a method of determining whether the blood pressure of a subject is sensitive to sodium intake. The method includes administering an epithelial sodium channel blocker and sodium chloride to the subject. The method also includes waiting for about 10 minutes or more. Further, the method includes determining at least one of systolic and diastolic blood pressure of the subject. The method also includes comparing the blood pressure to a control measurement or to a predetermined reference value. An increase in systolic and/or diastolic blood pressure compared to the control measurement indicates that the blood pressure of the subject is sensitive to sodium intake.

In some embodiments repeated sessions of the method are carried out. In one embodiment one session is carried out at least essentially without administration of an epithelial sodium channel blocker, for example by administering only NaCl or a composition that includes NaCl but no epithelial sodium channel blocker. In one embodiment in one session is at least essentially no epithelial sodium channel blocker is administered by administering a composition that essentially consists of NaCl but no epithelial sodium channel blocker. A further session is carried out with a combination of NaCl and an epithelial sodium channel blocker. Blood pressure values after administration of the combination of NaCl and the epithelial sodium channel blocker can then be compared to blood pressure values after administration of NaCl/composition without the epithelial sodium channel blocker. A significant increase in blood pressure values after administration of the combination, when compared to blood pressure values after administration of NaCl/composition but no epithelial sodium channel blocker, indicates that the blood pressure of the subject is sensitive to sodium intake.

In a fourth aspect the invention provides a kit of parts. The kit includes a first and a second container. The first container includes sodium chloride but is at least essentially void of an epithelial sodium channel blocker. The second container includes a combination of an epithelial sodium channel blocker and sodium chloride.

In some embodiments the first and the second container further include a monosaccharide such as for instance glucose.

In some embodiments the first container contains only sodium chloride and no further substance besides a fluid such as air. In some embodiments the first container contains essentially only sodium chloride. In some embodiments the second container contains only a monosaccharide such as for instance glucose, and no further substance besides a fluid such as air. In some embodiments the second container essentially contains only a monosaccharide. In some embodiments the second container contains only a combination of an epithelial sodium channel blocker and sodium chloride, and no further substance besides a fluid such as air.

### BRIEF DESCRIPTION OF THE DRAWINGS

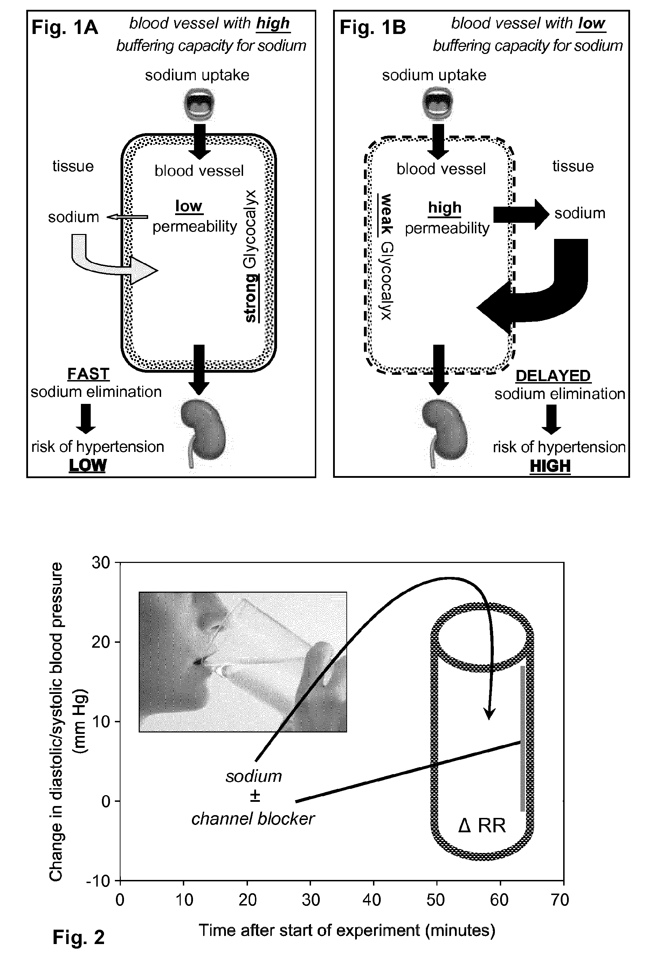

**Figure 1** illustrates, without being bound by theory, an assumption on the basis of a method according to the invention. **Figure 1A** depicts the situation of a blood vessel with an intact and strong endothelial glycocalyx, which is able to quickly buffer sodium in the blood. **Figure 1B** depicts the situation of a blood vessel with a weak endothelial glycocalyx, which has a limited buffering capacity for sodium in the blood.

**Figure 2** depicts an embodiment of a method and a use according to the invention. Following oral uptake of a combination of sodium chloride and an epithelial sodium channel blocker arterial blood pressure is determined. In the Examples blood pressure values were determined at 10 minutes intervals over a period of about 60 minutes. Δ RR indicates the change in blood pressure (Riva-Rocci measurement).

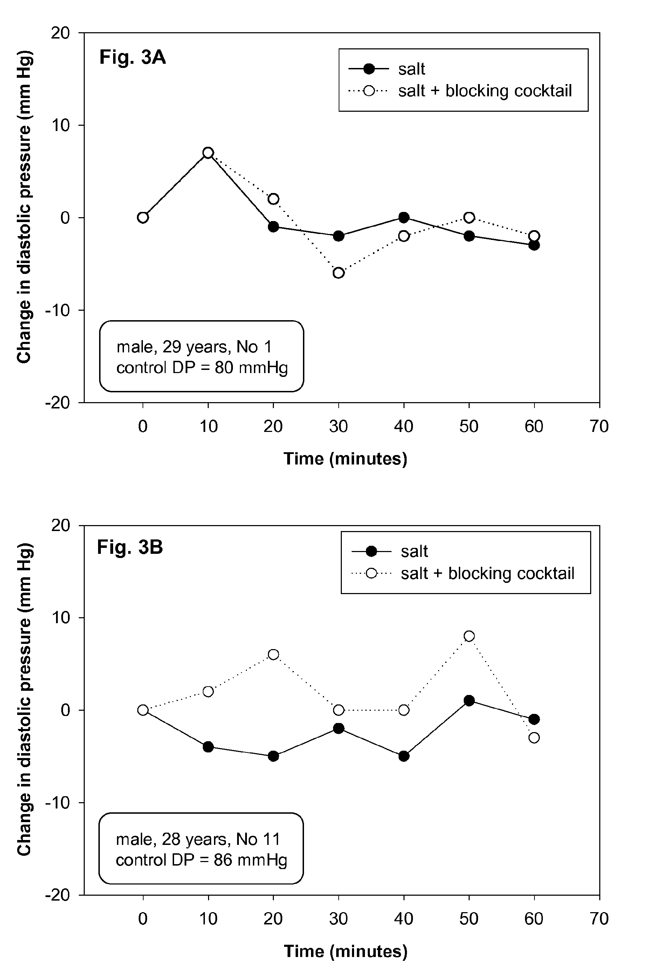

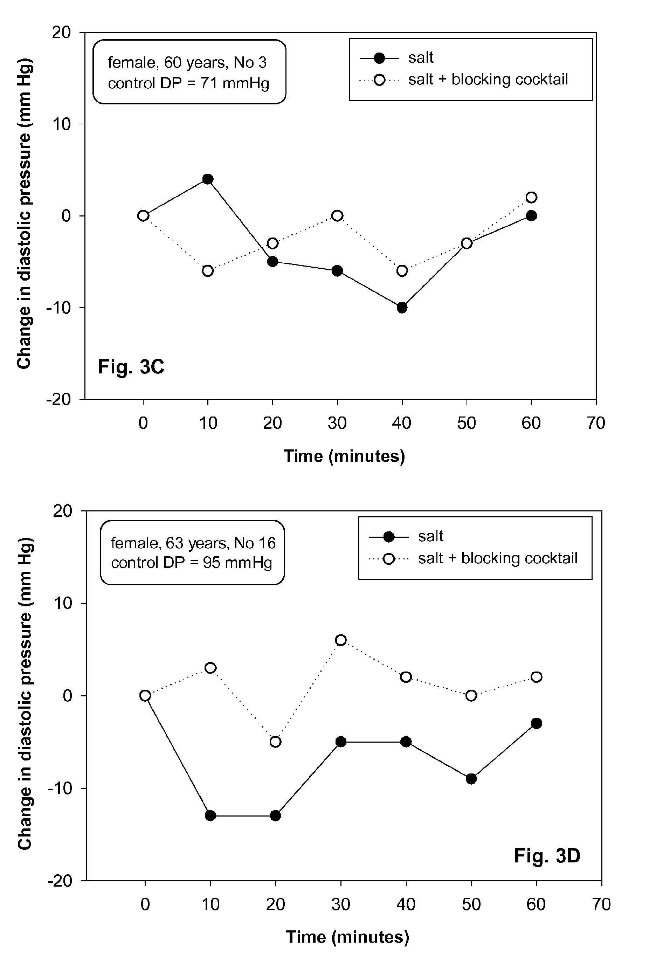

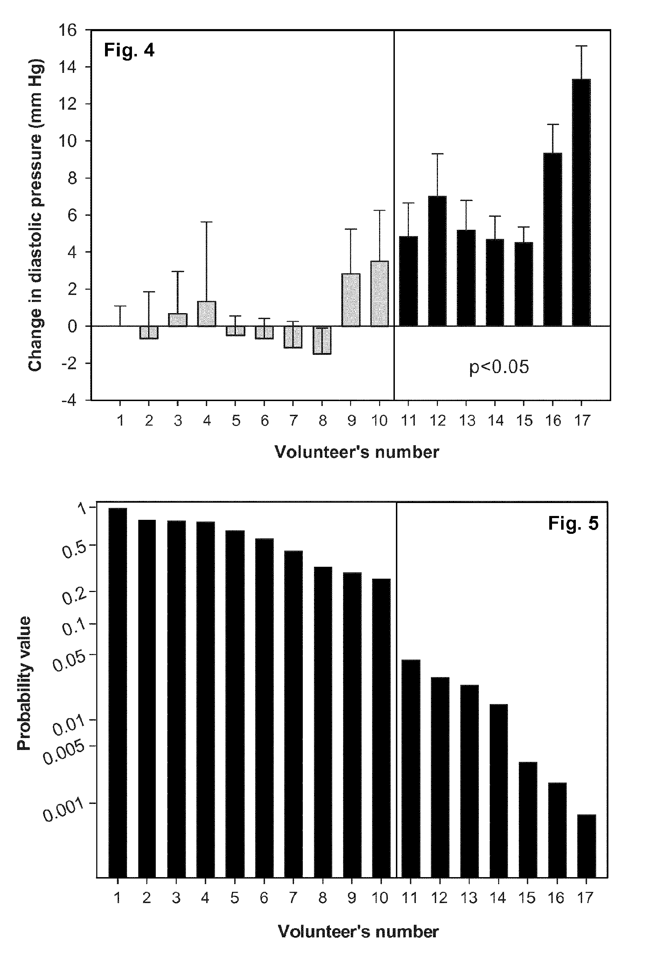

**Figure 3** depicts four representative examples (3A to 3D) of diastolic blood pressure takings of different volunteers after oral intake of a combination of sodium chloride, glucose, hydrochlorothiazide and the epithelial sodium channel blocker triamterene. The indicated number is the volunteer number also used in Fig. 4 and Fig. 5. Control DP = mean value of two diastolic blood pressure measurements taken at an interval of 2 minutes immediately before application of sodium/sodium channel blocker.

**Figure 4** depicts the changes of diastolic blood pressure in response to a combination of sodium chloride, glucose, hydrochlorothiazide and triamterene relative to a control measurement. 6 measurements were taken from each volunteer at intervals of 10 minutes in the course of an hour. Values at the left hand side up to volunteer 10 are statistically not significant, i.e. volunteers 1 to 10 are not salt sensitive. Increased values at the right hand side are statistically significant, i.e. the volunteers 11 to 17 are salt sensitive (cf. Fig. 5).

**Figure 5** depicts the probability value, which indicates the statistical significance of measurements. Arithmetic means at the left hand side up to volunteer 10 are statistically not significant, whereas means at the right hand side (volunteers 11 to 17) are statistically significant.

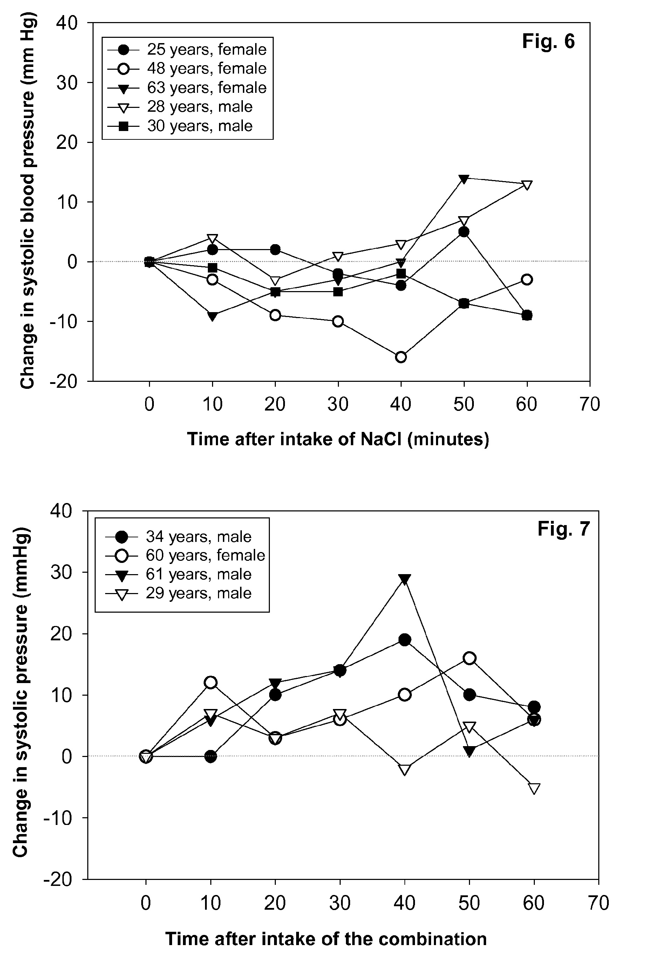

**Figure 6** depicts measurements of systolic blood pressure after oral intake of sodium chloride, glucose and hydrochlorothiazide, without the epithelial sodium channel blocker hydrochlorothiazide.

**Figure 7** depicts measurements of systolic blood pressure after oral intake of a combination of sodium chloride, glucose, hydrochlorothiazide and the epithelial sodium channel blocker triamterene.

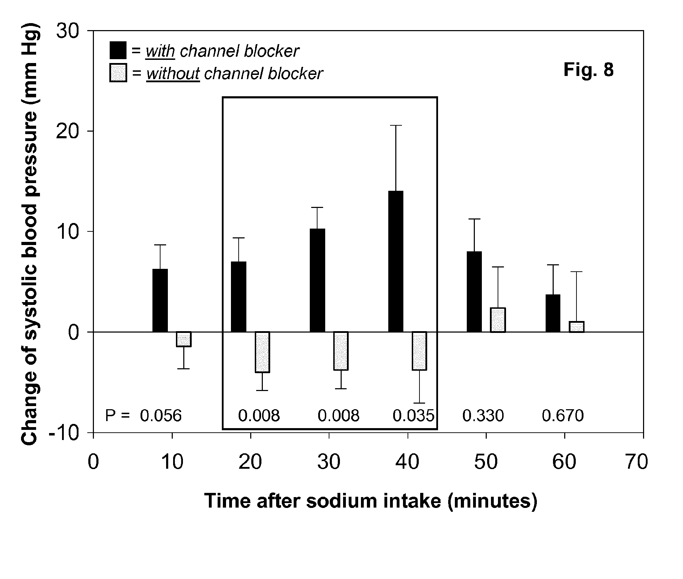

**Figure 8** depicts the mean values of the changes in systolic blood pressure with (black bars) and without (light bars) uptake of the epithelial sodium channel blocker.

### DETAILED DESCRIPTION OF THE INVENTION

The invention provides a combination of an epithelial sodium channel blocker and sodium chloride. In some embodiments the combination includes sodium chloride in an amount of about 1 g or more. The amount of sodium chloride may also be about 1.5 g or more, such as about 1.75 g or more, about 2 g or more, about 2.25 g or more, about 2.5 g or more, about 2.75 g or more, about 3 g or more, about 3.25 g or more, or about 3.5 g or more. In some embodiments about 3.75 g or more of sodium chloride are included in a combination of the invention. A sodium chloride amount of about 4 g or more may also be included in a combination of the invention. In some embodiments a combination of the invention contains about 4.25 g or more of sodium chloride. The amount of sodium chloride is in some embodiments about 4.5 g or more or about 4.75 g or more. The combination of the epithelial sodium channel blocker and sodium chloride may be included in a single pharmaceutical composition or in an aqueous medium. In some embodiments the epithelial sodium channel blocker and sodium chloride are provided as a mixture of solid substances. In some embodiments the combination is provided in the form of an aqueous solution.

In embodiments where the combination of the epithelial sodium channel blocker and sodium chloride is provided as an aqueous solution, the concentration of sodium chloride in the solution may in some embodiments be about 12.5 g/l or more, such as about 15 g/l or more, about 18 g/l or more or about 20 g/l or more. In some embodiments about 22 g/l or more of sodium chloride are included in a solution that includes the combination of the invention. About 25 g/l sodium chloride or more may be included in a solution of a combination of the invention. In some embodiments about 28 g/l NaCl or more are present in a solution with a combination of the invention. A concentration of sodium chloride of about 30 g/l or more may also be included in a respective solution. In some embodiments a solution is provided that includes a concentration of sodium chloride of about 32 g/l, about 35 g/l, about 38 g/l, about 40 g/l, about 45 g/l, about 50 g/l, about 55 g/l, about 60 g/l, about 65 g/l, about 70 g/l, about 75 g/l, about 80 g/l, about 85 g/l, about 90 g/l, about 95 g/l, about 100 g/l, about 110 g/l, about 120 g/l, about 130 g/l, about 140 g/l, about 150 g/l, about 160 g/l, about 170 g/l, about 180 g/l, about 190 g/l, about 200 g/l or more.

The amount of sodium channel blocker included in the combination depends on the epithelial sodium channel blocker used. Any compound may be used that is physiologically acceptable and that is capable of inhibiting epithelial sodium channels. Generally a compound is used that reversibly inhibits epithelial sodium channels. The epithelial sodium channel blocker is typically an endothelial sodium channel blocker in that it inhibits the function of sodium channels of the endothelial layer of cells lining the interior surface of blood vessels. Examples of suitable compounds include, but are not limited to, triamterene, amiloride and benzyl amiloride (benzamil). An example of an irreversible inhibitor of endothelial sodium channels is phenamil. Benzamil and phenamil have substituted benzyl and phenyl groups, respectively, on the terminal nitrogen atom of the guanidino moiety of amiloride (Fig. 1) and have been found to be more potent than amiloride. In some embodiments sodium channel blocker exists in form of a free base and in the form of a salt such as a hydrochloride.

In some embodiments the combination of the invention includes triamterene in an amount of about 50 mg or about 75 mg, or more. In some embodiments triamterene is included in an amount of about 100 mg or more. In some embodiments the combination contains about 125 mg triamterene or more. An amount of about 150 mg triamterene or more may also be present. In some embodiments the combination includes triamterene in an amount of about 200 mg, about 250 mg, about 300 mg, about 325 mg, about 350 mg or more. In some embodiments the combination of the invention includes amiloride in an amount of about 7.5 mg or more. In some embodiments about 10 mg or more of amiloride are included in the combination. In some embodiments about 12.5 mg or more of amiloride, such as about 15 mg or more are included in the combination. In some embodiments the combination includes triamterene in an amount of about 17.5 mg or more, such as about 17.75 mg or about 18 mg. In some embodiments the combination contains an amount of about 18 mg or more of triamterene. The combination may also include triamterene in an amount of about 19 mg, about 20 mg, about 25 mg, about 30 mg or more. In some embodiments the combination of the invention includes benzamil in an amount of about 2 mg or more, such as about 2.25 mg or more. In some embodiments the combination includes benzamil in an amount of about 2.5 mg or more. The combination may also include about 2.75 mg or more of benzamil. In some embodiments the combination contains about 3 mg, such as about 3.25 mg, about 3.5 mg or more of benzamil. In some embodiments about 4 mg or more of benzamil are included in the combination. The combination may for instance also include about 4.5 mg, about 5 mg, about 5.5 mg, about 6 mg or more of benzamil. It is understood that the epithelial sodium channel blocker such as triamterene, amiloride or benzamil may be combined with sodium chloride in any illustrative amount in terms of g as exemplified in paragraph 29 above. Triamterene is commercially available under the trade name Dyrenium® amiloride is commercially available under the trade name Midamor®.

In embodiments where the combination of the epithelial sodium channel blocker and sodium chloride is provided as an aqueous solution, the concentration of triamterene in the solution may in some embodiments be about 12.5 g/l or more, such as about 2 mg/l. In some embodiments triamterene is present in a concentration of about 4 mg/l. A concentration of about 8 mg/l triamterene may also be present. The concentration of triamterene is in some embodiments about 10 mg /l. In some embodiments a concentration of about 12 mg /l triamterene is included in a respective solution. In some embodiments triamterene is included in a concentration of about 15 mg/l or more, of about 17 g/l, about 20 g/l, about 22 g/l, about 25 g/l, about 27 g/l, about 30 g/l, about 32 g/l, about 35 g/l, about 37 g/l, about 40 mg/l, about 48 mg/l, about 55 mg/l, about 60 mg/l, about 65 mg/l, about 70 mg/l about 75 mg/l, about 80 mg/l about 85 mg/l or more. In some embodiments triamterene is included in a concentration of about 100 mg/l or more. If in a respective aqueous solution amiloride or benzamil are provided, amiloride or benzamil may for instance be included in a concentration of about 5 mg/l or more, such as about 10 mg/l or more. In some embodiments about 20 mg/l or more of amiloride and/or benzamil may be present. About 30 mg/l or more, including about 40 mg/l, about 50 mg/l, about 60 mg/l, about 70 mg/l, about 80 mg/l, about 90 mg/l, about 100 mg/l, about 110 mg/l, about 120 mg/l, about 130 mg/l, about 140 mg/l, about 150 mg/l, about 160 mg/l, about 170 mg/l, about 180 mg/l, about 200 mg/l, about 250 mg/l, about 300 mg/l, about 350 mg/l, or about 400 mg/l or more. In some embodiments amiloride or benzamil are included in a concentration of about 500 mg/l or more. A concentration of about 520 mg/l or more, including about 550 mg/l amiloride or benzamil is in some embodiments chosen. In some embodiments a total concentration of about 570 mg/l or more of amiloride and/or benzamil is present. The concentration of amiloride and/or benzamil may also be about 600 mg/l or more. In some embodiments a concentration of about 650 mg/l or more of amiloride and/or benzamil is provided in a solution that contains a combination of the invention. In some embodiments about 700 mg/l or more of amiloride and/or benzamil are present. About 750 mg/l or more of amiloride and/or benzamil may also be included in a respective solution. Amiloride and/or benzamil may be included in a concentration of about 800 mg/l or more. In some embodiments amiloride or benzamil are included in a concentration of about 900 mg/l, about 1 g/l, about 2 g/l, about 5 g/l, about 10 g/l, or more. The epithelial sodium channel blocker such as triamterene, amiloride or benzamil may be combined with sodium chloride in any illustrative amount in terms of g/l as exemplified in paragraph 30 above.

In some embodiments the combination is formulated for oral application. It may for example be included in a pharmaceutical composition for oral application. In some embodiments the combination is formulated for a single application to a subject.

The present invention is based on the surprising finding that the functional state of the endothelial glycocalyx (eGC) is a prognostic parameter for a disposition of hypertension. The term "hypertension" as used herein refers to high arterial blood pressure. Generally high arterial blood pressure is characterized by a systolic blood pressure that is consistently over 140 mm Hg and/or a diastolic blood pressure that is consistently over 90 mm Hg. If either or both of the systolic blood pressure and the diastolic blood pressure are too high, a subj ect has hypertension.

Over the past years clinical detection methods such as the method of pulse wave analysis (Gurovich AN & Braith RW, Hypertens Res (2011) 34, 166-169) or various imaging technologies (e.g. intima media thickness) have been refined to a degree that the structural /functional state of the vessel system can be conceived in detail. Apart from the relative complexity of these examinations, which does not permit a widespread use throughout the population for purely preventive purposes, these modern methods do only detect the state of the blood vessels once already visible (structural) changes have occurred. Therefore, these methods are not suitable for the early diagnosis of a disposition for hypertension.

Current data on humans and mammals in general indicate that the cause of hypertension may be directly linked to the uptake/excretion of sodium chloride and water. The daily sodium intake in industrial countries exceeds physiological needs to a high degree. While for millions of years the evolutionary ancestors of modern man had a diet containing less than 1 g salt/day, present consumption is in the range of about 10 g salt/day. It is generally accepted that the high amount of sodium consumed via modern nutrition (rich in salt), including finished products, often overburdens the excretion capacity of the kidneys. As a result sodium is kept and stocked in the body and in the course of years blood vessels (Oberleithner, H., et al., Proc Natl Acad Sci U S A (2007), 104, 16281-16286) damages to organs such as the brain, kidneys and the heart (Oberleithner, H, & de Wardener, H.E., Blood Purif (2011) 31, 82-85). The exact mechanism linking high sodium intake and its deleterious effect in the body is, however, unknown. Though efforts are made worldwide to limit sodium consumption (see WHO report: *http:llwww.who.int*/*dietphysicalactivity*/*reducingsalt*/*en*/ *index.html*), but this development (reduction of the everyday sodium consumption by at least 50%) will occur only very slowly, if at all.

As is well known in the art, the inner wall of blood vessels is lined with a layer of endothelial cells, which are of high importance for the function of blood vessels. On this endothelial surface there is a so-called endothelial glycocalyx (eGC), an anionic glycoprotein layer of about 500 nm in thickness and rich in water. The eGC participates in the regulation of vascular permeability, in the control of flow- and pressure-induced mechanotransduction of the endothelium and may play a crucial role in the pathogenesis of inflammation. Proteoheparan sulphate macromolecules, anchored in the plasma membrane expose negatively charged glycosaminoglycan side chains with binding sites for inorganic cations. This eGC is capable of temporarily buffering sodium of the blood. Explanations for the inventor's findings can be taken from recent research results, which support the view that the functional state this eGC has a determining influence on the transport of sodium from the blood stream into tissue (Oberleithner, H., et al., Pflügers Arch (2011) 462, 519-528). In other words, an intact eGC defines a functional barrier for the resorption of sodium ions that have entered the blood. The ions can thus be eliminated via the kidneys and do not have to take a route through the entire organism, involving a transient deposit in tissues and organs (cf. Fig. 1). The eGC may play a prominent role as a buffer barrier for sodium.

In a previous *in vitro* study (Oberleithner et al., 2011, supra) the eGC sodium store for endothelial monolayers, grown under low (135 mM) extracellular sodium conditions was measured to be 16 nmol/cm². When this measurement is extrapolated to man, assuming that the total endothelial surface is 350 m² and the glycocalyx thickness is 300 nm, then about 0.7 g of sodium (about 35 mmol) can be calculated for the total amount of sodium being retained by the negative charges of the eGC. This storage capacity is reduced by about two thirds (about 12 mmol) after sodium excess (i.e. exposure of the *ex vivo* endothelium to 150 mM sodium for 5 days). While these calculations are based on *in vitro* experiments, where the glycocalyx is less well developed than *in vivo,* they may provide a partial explanation for the inventor's findings.

Without being bound by theory, for a better understanding on a hypothetical basis, it is assumed that the use of the combination of the invention and a method according to the invention can be envisaged to have a basis as follows. Orally taken sodium enters the blood stream. In case the eGC is intact and the body's buffering capacity is high, sodium is temporarily being bound to the Glycocalyx, in an osmotically inactive form. Blood pressure remains essentially unaffected. In the eGC is developed insufficiently, whether genetically inhered or acquired during life, and the body's buffering capacity is low, sodium is - being buffered insufficiently - circulating as an osmotically active species and binding water. Blood pressure rises temporarily.

The present inventor assumes that the degree of change in blood pressure quantitatively correlates with the salt sensitivity or the disposition of a subject for hypertension.

As has only recently been discovered, endothelial cells have so-called sodium channels, which are involved in the fast removal of sodium from blood into tissue. Accordingly the present inventor assumes, without being bound by theory, that two functional barriers exist for sodium, the eGC and the actual endothelium with sodium channels. In order to be able to characterize the eGC it is thus necessary to temporarily disable the channels. Therefore, the oral absorption of sodium chloride needs to be combined with the blockade of the sodium channels. This can be achieved by concomitant absorption of NaCl in an amount of roughly about 1 g or more and an epithelial sodium channel blocker.

In some embodiments the combination of NaCl and the epithelial sodium channel blocker further includes a monosaccharide. Many monosaccharides are known in the art. In some embodiment the monosaccharide is a hexose. As illustrative examples glucose (also called dextrose, corn sugar or grape sugar), fructose (also called fruit sugar), galactose and mannose are named here as a hexose monosaccharide. In some embodiments a combination of any two or more hexose monosaccharides may be included in a combination of the invention. In some embodiment the monosaccharide is a pentose. Examples of a pentose monosaccharide that may be included in a combination of the invention include, but are not limited to, arabinose, ribose, and xylose, as well as combinations thereof. In some embodiments the combination of NaCl and the epithelial sodium channel blocker further includes a disaccharide. Examples of a disaccharide include, but are not limited to, sucrose (saccharose), lactose and maltose, as well as combinations thereof.

The mono- or disaccharide may in some embodiments be present in the combination in a molar amount roughly in the range from about one third or about half, including about 0.65-fold of the amount of sodium chloride to about equal to the amount of sodium chloride. In other words, in such embodiments sodium chloride may be present in the combination in a molar amount roughly in the range from a value about equal to the amount of the mono- or disaccharide to about three-fold or two-fold of the amount of the mono- or disaccharide. In some embodiments including 1.5-fold of the amount of the mono- or disaccharide. As an illustrative example, in some embodiments a combination of the invention may be provided that includes a mass of 3.5 g (about 0.06 mol) sodium chloride. In such embodiments glucose or fructose may be present in the combination in a mass amount roughly in the range from about 3.6 g (about 0.02 mol) or about 5.4 g (about 0.03 mol) to about 10.8 g (about 0.06 mol), including about 7.2 g (about 0.04 mol).

In some embodiments a mono- and/or a disaccharide is included in a combination of the invention in an amount of about 0.2 g or more, such as of about 0.3 g or more. In some embodiments an amount of about 0.5 g or more of a mono- and/or disaccharide is included in the combination. An amount of about 0.75 g or more of a mono- and/or disaccharide may also be selected. In some embodiments the combination includes about 1 g or more, including about 1.2 g or more of a mono- and/or disaccharide. As an illustrative example, a total amount of 1.1 g of glucose and/or fructose may be present. In some embodiments an amount of about 1.4 g or more, about 1.6 g or more, about 1.8 g or more, about 2 g or more, about 2.5 g or more, about 3 g or more, about 3.5 g or more, or about 4 g or more of a mono- and/or disaccharide is included in the combination In some embodiments the amount of a mono-and/or disaccharide in the combination is about 4.5 g or more. In some embodiments about 5 g or more of a mono- and/or disaccharide is present in the combination. In some embodiments the combination includes about 5.5 g or more of a mono- and/or disaccharide. About 6 g or more of a mono- and/or disaccharide may also be included in a combination. In some embodiments about 6.5 g or more of a mono- and/or disaccharide are provided in the combination. In some embodiments the amount of a mono- and/or disaccharide in the combination is about 7 g or more, about 7.5 g or more, about 8 g or more, about 8.5 g or more, or about 9 g or more. It is understood that the mono- or disaccharide may be combined with sodium chloride in any illustrative amount in terms of g as exemplified in paragraph 29 above. It may further be combined with an epithelial sodium channel blocker in any illustrative amount in terms of mg/l or g/l as exemplified in paragraph 32 above.

In embodiments where the combination is provided in the form of an aqueous solution, in some embodiments the concentration of the mono- and/or disaccharide may be about 3 g/l or more, such as about 4 g/l. In some embodiments a mono- and/or disaccharide is present in a concentration of about 5 g/l. In some embodiments a concentration of about 6 g/l or about 7 g/l of a mono- and/or disaccharide is included in the combination. In some embodiments the concentration of mono- and/or disaccharide is chosen to be about 8 g/l. As an illustrative example about 8 g/l of a hexose may be present. The concentration of a mono-and/or disaccharide may in some embodiments be about 10 g/l, about 12 g/l or about 14 g/l. In some embodiments about 16 g/l of mono- and/or disaccharide are provided. The concentration of a mono- and/or disaccharide may also be about 18 g/l, about 20 g/l, about 22 g/l, about 25 g/l, about 30 g/l, about 35 g/l, about 40 g/l, about 45 g/l, about 50 g/l, about 55 g/l, about 60 g/l, about 65 g/l, about 70 g/l, about 75 g/l, about 80 g/l, about 85 g/l, about 90 g/l, about 100 g/l, about 110 g/l, about 120 g/l, about 130 g/l, about 140 g/l, about 150 g/l, about 160 g/l, about 170 g/l, about 180 g/l, about 190 g/l, about 200 g/l, about 210 g/l, about 220 g/l, about 230 g/l, about 240 g/l, about 250 g/l, about 260 g/l, about 270 g/l, about 280 g/l, about 290 g/l, about 300 g/l, about 320 g/l, about 350 g/l, or more. The mono- or disaccharide may be combined with sodium chloride in any illustrative amount in terms of g/l as exemplified in paragraph 30 above. It may further be combined with an epithelial sodium channel blocker in any illustrative amount in terms of mg/l or g/l as exemplified in paragraph 33 above.

Providing a monosaccharide or disaccharide in a combination of the invention may be advantageous to achieve a quick resorption of administered sodium in a use or a method according to the present invention. Sodium is being resorbed in the intestine together with glucose (in a molar ratio of Na⁺ : glucose =2:1). In some embodiments resorption of sodium chloride can be accelerated by concurrent absorption of a mono- or disaccharide, in particular glucose.

In some embodiments the combination of NaCl and the epithelial sodium channel blocker includes a thiazide diuretic and/or a thiazide-like diuretic. Illustrative examples of a thiazide and a thiazide-like diuretic include, but are not limited to, hydrochlorothiazide, bendroflumethiazide, chlortalidone, and xipamide. A combination of triamterene and hydrochlorothiazide is commercially available under the trade names Dyazide® and Maxzide®. A combination of amiloride and hydrochlorothiazide is commercially available under the trade names Apo-Amilzide®, Gen-Amilazide®, Moduret®, Moduretic®, Nu-Amilzide® and Dytide® H. In some embodiments the combination of NaCl and the epithelial sodium channel blocker includes a loop diuretic. Illustrative examples of a loop diuretic include, but are not limited to, furosemide, bumetamide, piretanide and torasemide. It is noted in this context that triamterene and amiloride, which may be used as epithelial sodium channel blockers in the combination of the invention, are being classified as potassium sparing diuretics. This classification is due to their inhibition of sodium channels, which is effective in the tubule of the kidney as well. However, as also explained below, the uses and methods according to the present invention are based on the action of such compounds on the sodium channels of vascular endothelial cells, i.e. cells that are in direct contact with blood.

In conjunction with a combination as defined above the present invention also provides a method and a use for diagnosis or for stratifying the risk of disease. A combination of NaCl and an epithelial sodium channel blocker as defined above may be used in diagnosis or prognosis assessment of hypertension and/or a hypertension related condition in a subject, generally a mammalian subject. A respective use or method can provide an indication of a risk of developing hypertension in a subject. The use or method may thus serve in assessing propensity for hypertension and/or a hypertension related condition or screening subjects for a propensity for hypertension and/or a hypertension related condition. In this regard the term "hypertension" refers to arterial hypertension. The respective combination may also be used to determine whether the blood pressure of a subject is sensitive to sodium intake. A positive assessment in this regard will generally be an indication that the vascular system of the respective subject is sensitive to changes in sodium intake. Further, the combination may also be used in stratifying a subject for hypertension prevention measures or for hypertension therapy. An "individual" or "subject" as used herein refers to any mammal, including e.g. a rabbit, a mouse, a Guinea pig, a hamster, a dog, a pig, a cow, a sheep, a horse, a macaque, a gibbon and a human. A respective mammal may in some embodiments be a veterinary animal such as a farm animal, a domestic animal or laboratory animal. Where the subject is a human, the subject may be a patient. A method employing a combination according to the invention may in some embodiments be a method for risk assessment, a method for diagnosis or prognosis of the occurrence of hypertension and/or a hypertension related condition in a subject. In some embodiments a method or use according to the invention relates to the assessment of prehypertension in a subject, i.e. a subject with a systolic blood pressure of values in the range from about 120 to about 139 mm Hg and/or a diastolic blood pressure of values in the range from about 80 to about 89 mm Hg.

The term "risk stratification" according to the invention generally includes identifying subjects having a prognosis of developing hypertension, subjects having a prognosis of exacerbation in hypertension, as well as identifying subjects having a low, a medium and a high risk of developing hypertension. The term includes finding subjects having a blood pressure that is sensitive to sodium intake. The term includes finding healthy subjects whose blood pressure may rise to hypertension levels in the future. The term also includes finding subjects suffering from hypertension and whose blood pressure is sensitive to sodium intake. In this regard the method of the invention may be useful in both diagnosis and treatment. Measures may for example be taken to reduce the subject's sodium amount in the body, for example to increase sodium clearance and/or to reduce sodium intake. Stratification may be based on the probability (or risk) of developing or of exacerbation of hypertension. A method or use according to the invention may also serve in stratifying the probability of the risk of any given cardiovascular disease or the risk of any given cardiac or cardiovascular event for a subj ect.

A large variety of hypertension related disorders are known in the art. Hypertension is known to result in damage and/or disorders of various organs, in particular of the heart, the brain, the kidney and the retina of the eye. In some embodiments a hypertension related condition is a cardiovascular disease. A risk of a condition to which a method or use according to the invention relates (supra) includes, but is not limited to, a risk of atherosclerosis, of coronary atherosclerosis, of vascular stenosis, of thrombosis, of peripheral vascular disease, including peripheral artery disease, of stroke, of haemorrhagic stroke, of ischemic stroke, of ischemic heart disease, of congestive heart failure, of Angina pectoris, myocardial infarction of ventricular arrhythmias, of myocardial ischemia, of coronary heart diseases, of acute coronary syndrome, of (acute) myocardial infarct, of cardiac insufficiency, of angina pectoris, of renal artery stenosis or of renal nephrosclerosis. "Ischemic heart disease" or myocardial ischemia as used herein is understood as referring to a disease characterized by reduced blood supply to the heart muscle, usually due to coronary artery disease (atherosclerosis of the coronary arteries). A "stenosis" as used herein, is defined as an abnormal narrowing in a blood vessel or other tubular organ or structure. "Cardiac insufficiency" as used herein refers to as an acute or chronic inability of the heart to supply sufficient blood to the tissue, and as a result, oxygen, to guarantee tissue metabolism at rest and under stress. Clinically, cardiac insufficiency is present, when typical symptoms (dyspnea, fatigue, liquidity retention) exist, the cause of which is based on a cardiac dysfunction within the meaning of a systolic or diastolic dysfunction.

In some embodiments a method of the invention includes taking a control measurement. The control measurement is a measurement subsequently used to a measurement taking after administration of the combination of NaCl and the epithelial sodium channel blocker. In some embodiments the control measurement is taken within a time interval of an hour or less, including 10 minutes or less, or 2 minutes or less. In some embodiments a plurality of control measurements is taken such as two control measurements or three control measurements or more. The blood pressure value(s) of the control measurement(s) may be recorded. The control measurement or measurements is/are the measurement of the (arterial) blood pressure of the subject. While both systolic and diastolic blood pressure measurements may be carried out, generally the same type of blood pressure measurement is carried out that is also carried out after administration of the combination of NaCl and the epithelial sodium channel blocker. Hence, where after administration of the combination the diastolic blood pressure is to be determined, in the control measurement diastolic blood pressure is generally likewise determined. In some embodiment a control value for subsequent blood pressure measurements is determined as the average, e.g. the arithmetical average or the mean, of several, e.g. two or three, blood pressure measurements taken before administration of a combination according to the invention.

In the context of the present invention blood pressure may be determined according to any desired method at any one or more arteries of a subject. In typical embodiments peripheral blood pressure is determined, for example at one or more limbs of the subject. In some embodiments arterial blood force, i.e. the pressure applied by blood to the arterial wall, is determined. In some embodiments a blood pressure measurement of a human may for instance be carried out in the form of the indirect method first described by von Riva Rocci Recklinghaus. In this method an inflatable cuff is placed on the middle third of the upper arm and the pressure within the cuff is quickly raised up to complete cessation of circulation below the cuff, typically by inflating the cuff to a predetermined level. An electronic blood pressure measuring device or a reservoir of mercury at the end of a vertical glass column, in combination with listening to the artery just below the cuff with a stethoscope, may be used to determine the blood pressure value. In some embodiments an automatic, typically non-invasive, blood pressure gauge based on e.g. a pressure sensor, for example in combination with a press mechanism and/or a flow sensor and a sphygmomanometer, may be used.

A method of the invention may also include providing the combination of NaCl and the epithelial sodium channel blocker. The respective combination may for example be presented to the subject in the form of a powder or of an aqueous solution. In some embodiments a powder is provided that is then being stirred in water or in an aqueous medium. In some embodiments a combination according to the invention may be provided in the form of a stock solution, i.e. in the form of a solution that is to be diluted before use. Further, as mentioned above, the combination is administered to a subject. Administration is typically oral administration. In a method or use according to the invention the amount of the combination as defined above for administration to the subject is typically selected in such a way that a total amount of sodium chloride in the range from about 1 g to about 10 g is being administered, such as from about 2 g to about 8 g. In some embodiments the total amount of sodium is from about 2 g to about 6 g A sodium amount may also be chosen from about 3 g to about 8 g or about 4 g to about 8 g. In some embodiments an amount of sodium from about 4 g to about 6 g, including an amount of about 3.5 g, about 4.5 g, 5 g or about 5.5 g is chosen. Further, the amount of the combination as defined above for administration to the subject is typically selected in such a way that a total amount of the monosaccharide and/or disaccharide that is being administered is in the range from about 5 g to about 15 g. The total amount of the monosaccharide and/or disaccharide may also be from about 5 g to about 13 g. In some embodiments the amount of monosaccharide and/or disaccharide is from about 6 g to about 13 g, including from about 6 g to about 12 g. The combination may also include a total amount from about 6 g to about 10 g of monosaccharide and/or disaccharide. In some embodiments the total amount of the monosaccharide and/or disaccharide is from about 5 g to about 10 g, such as from about 6 g to about 9 g, about 7 g to about 10 g or about 7 g to about 9 g, including an amount of about 5.5 g, about 6.5 g, 7.5 g or about 8.5 g.

A respective method typically also includes waiting for a period of time, generally about 10 minutes or more. This waiting period generally occurs after administering the combination, such that sodium chloride is allowed to enter the blood stream of the subject. A method of the invention further includes measuring the blood pressure of the subject. As noted above, measuring the blood pressure may be done before administering a combination according to the invention. In this case the measurement is generally a control measurement. One or more measurements of the blood pressure are usually done after administering a combination according to the invention. A respective blood pressure measurement may accordingly be repeated. Such a measurement, carried out after administration of the combination, may be a measurement of the systolic blood pressure, a measurement of the diastolic blood pressure or both. Where a plurality of blood pressure measurements is carried out, the blood pressure measurements may be taken, at least substantially, after unitary time intervals or after varying time intervals. In some embodiments repeated determination of the subject's blood pressure is carried out at intervals of a certain, e.g. predetermined, length. The time interval between repeated measurements of the blood pressure may in some embodiments be selected in the range from about a minute to about 30 minutes. In some embodiments a period of about two minutes to about 30 minutes between measurements is chosen. In some embodiments the period is from about two minutes to about 25 minutes, such as from about three minutes to about 20 minutes. The period between measurements may also be in the range from about four minutes to about 20 minutes or from about five minutes to about 15 minutes. As an example a total amount of about 2 to 7 g NaCl together with an epithelial sodium channel blocker may have been administered and repeated measurements with a time interval from about five minutes to about 15 minutes between them may be done. In some embodiments a period from about five minutes to about 10 minutes or from about three minutes to about 10 minutes between measurements is chosen. An interval of e.g. four minutes or about five minutes may be chosen., As a few further examples, a period of about six minutes, about seven minutes, about eight minutes, about nine minutes, about ten minutes, about eleven minutes, about twelve minutes, about thirteen minutes, about fourteen minutes or about fifteen minutes may be chosen. After a combination according to the invention has been applied to a subject, the subject's arterial blood pressure may temporarily increase. The extent of this increase may in some embodiments be used to assess the degree of an increased risk of developing hypertension and/or for stratifying the risk of developing hypertension.

Where a plurality of blood pressure measurements is taken in a method according to the invention, the total time period during which this plurality of blood pressure measurements is taken may be selected as desired. In some embodiments taking blood pressure measurements is carried out over a period from about 5 minutes to about five hours. In some embodiments time interval from about 10 minutes to about four hours. As an illustrative example, a total amount of sodium chloride in the range from 1.5 to about 6 g NaCl may have been administered to the subject, and blood pressure may be measured over a period from about 10 minutes to about four hours. Measurements may also be carried out over a period from about 10 minutes to about three hours. In some embodiments blood pressure measurements are taken over a period from about 15 minutes to about four hours. In some embodiments the period is from about 20 minutes to about three hours. The time interval may also be selected in the range from about 20 minutes to about two hours. In one embodiment a solution of NaCl and a monosaccharide may have been administered and blood pressure measurements be done over a period from about 25 minutes to about 2.5 hours. The period may in some embodiments be from about 30 minutes to about two hours, such as from about 30 minutes to about 1.5 hours. The period may also be selected in the range from about 45 minutes to about 1.5 hours. In some embodiments a period of about 12 minutes, about 35 minutes, about 40 minutes, about 50 minutes, about 60 minutes, about 80 minutes or about 100 minutes is chosen.

In some embodiments a method as described above is repeated after a period of one or more days, such as after two, three, four, five, six, seven, eight days or more. The repeated performances of the method may be carried out independent from one another. Hence, time intervals or combinations used may be independently selected. In some embodiments the repeated performances of the method may be carried out with exactly identical settings. Typically the blood pressure measurements are carried out on the same blood pressure type in the repeated instances of carrying out the invention. Hence, where systolic blood pressure has been measured at the first session, systolic blood pressure will generally be measured again at the following and any further sessions that are carried out. If diastolic blood pressure has been measured at the first session, diastolic blood pressure will generally be measured again at the following and any further sessions that are carried out. Where both systolic and diastolic blood pressure have been determined in a first session, systolic blood pressure, diastolic blood pressure or both may be determined during a subsequent session.

In some embodiments where a plurality of sessions of a method according to the invention are being carried out, in each session a plurality of blood pressure takings are been carried out. In some of these embodiments with repeated blood pressure takings these repeated measurements of blood pressure are taken at at least essentially identical time intervals. As an illustrative example a method of the invention may be carried out twice with identical intervals of measuring blood pressure. The first blood pressure measurement after administration of the combination may in both sessions be taken after fifteen minutes, whereafter repeated blood pressure measurements may for example be taken six times at intervals of eight minutes. In such an embodiment blood pressure measurements are - at different days - taken at identical time points, when calculated from administration of the combination. Such a procedure allows a direct comparison of the values taken during the two or more sessions. The present inventor has found that it is advantageous to apply such embodiments and to calculate the average of the corresponding blood pressure measurements, i.e. calculate the average of values taken after fifteen minutes, after 23 minutes, after 31 minutes etc. following administration of the combination of the invention.

In some embodiments where a plurality of sessions of a method according to the invention is being carried out, one session may serve as a reference session. This session may be carried out in the same way, i.e. using the same time intervals as another session. A respective reference session may be a session where a method according to the invention is being carried out without administration of an epithelial sodium channel blocker. Accordingly, a first session and a second session may be carried out. The first and the second session may be based on at least essentially identical time intervals used. In the first session a combination as defined above is applied to a subject and blood pressure measurements are performed as described above. The combination includes an epithelial sodium channel blocker and sodium chloride. The combination may also include a mono- or a disaccharide. In the second session a substance or a combination of substances is used for administration that at least essentially only differs from the combination used in the first session in that it does not include significant amounts of an epithelial sodium channel blocker.

In this regard a significant amount of an epithelial sodium channel blocker is an amount that physiologically inhibits sodium channels in a subject after administration. Hence, sodium chloride or a combination of sodium chloride and a mono- or a disaccharide may for instance be applied in the second session. Blood pressure measurements are then performed as in the first session. Typically the first and the second session are carried out on different days. The order of carrying out the first and the second session may be selected as desired. In some embodiments the second session is carried out before the first session. Blood pressure values of corresponding time points of the second session may then be subtracted from the blood pressure values of the first session. As an illustrative example a blood pressure value taken fifteen minutes after administration of a substance or combination without significant amounts of an epithelial sodium channel blocker in the second session is subtracted from a blood pressure value taken fifteen minutes after administration of a combination that includes an epithelial sodium channel blocker in the first session. In some embodiments a plurality of first sessions and/or second sessions may be carried out. Average blood pressure values of individual time points of all first sessions and all second sessions may be determined and the mean of the second sessions be subtracted from the mean of the first sessions.

In some embodiments a method or use according to the invention consists of the steps (a) administering an epithelial sodium channel blocker and sodium chloride to the subject, (b) waiting for about 10 minutes or more (c) determining at least one of systolic and diastolic blood pressure of the subject, and (d) comparing the blood pressure to a control measurement or to a predetermined reference value. In some embodiments this use or method is repeated in the form of one or more further sessions, where the parameters of each session may be identical or different as explained above.

A kit according to the invention may include, including consist of, two or more containers. A first container includes sodium chloride but is at least essentially void of an epithelial sodium channel blocker. The container may further include a mono- or disaccharide such as glucose. A second container of the kit includes a combination of sodium chloride and an epithelial sodium channel blocker. Typically the second container includes the same matter as the first container with the only difference being that the second container includes the epithelial sodium channel blocker whereas the first container is at least essentially void thereof. The matter included in the containers may be provided in solid form, such as a powder, or it may be provided in the form of an aqueous solution. The kit may be used for carrying out a method according to the invention. The kit may be for prognosis, diagnosis and/or risk stratification of disease. The first container may be used for a session of the method of the invention that serves as a control measurement.

Additional objects, advantages, and features of this disclosure will become apparent to those skilled in the art upon examination of the following examples thereof, which are not intended to be limiting. Thus, it should be understood that although the present disclosure is specifically disclosed by exemplary embodiments and optional features, modification and variation of the disclosures embodied therein herein disclosed may be resorted to by those skilled in the art, and that such modifications and variations are considered to be within the scope of this disclosure.

### EXAMPLES

The following example serves in illustrating an embodiment of using a combination according to the invention. Reference can also be made to Fig. 2.

A test person orally takes a "cocktail" consisting of:
5 g NaCl, 100-150 mg Triamterene (or 10-15 mg Amiloride) and 7.5 g of glucose, dissolved in 200 ml of water. In order to allow the test person to quickly get rid of the salt taken up, 50 mg of a diuretic (Hydrochlorothiazide) is added to the cocktail. The combination of Triamterene (or Amiloride) and Hydrochlorothiazide is commercially available since a number of years in the form of tablets (manufactured by different pharmaceutical companies). In the present examples two tablets of Dytide® H (MIBE GmbH Arzneimittel, Germany) were administered to subjects.
Cocktail: 5 g NaCl, 7.5 g of glucose, 100 mg Triamterene (or 10 mg Amiloride), 50 mg Hydrochlorothiazide

### I: MEASUREMENT OF THE DIASTOLIC BLOOD PRESSURE

The test was carried out on 17 test persons (at an age from 25 to 63 years). Each test person underwent two sessions of 1 hour each. During the first session the salt/sugar cocktail (without channel blocker) was taken up and subsequently the blood pressure was measured at intervals of 10 minutes. In the second session (at least 2 days later) the salt/sugar cocktail (with channel blocker) was taken up and subsequently the blood pressure was measured at intervals of 10 minutes. The change in *diastolic* blood pressure in relation to the first control measurement was evaluated. As a control served an averaged measurement, namely two blood pressure measurements, taken at an interval of two minutes immediately before intake of the cocktail.

Two curves result from the two measurements (salt ± blocking cocktail). Four examples are shown in Fig. 3 (A-D). The more the curves deviate from each other, the more prominent is the salt sensitivity of the test person's blood pressure. This appears independent of the source blood pressure and from the sex of the experimental subj ects.

The averages (± S.E.M.) of the six measurements in each case from a total of 17 test persons are depicted in Fig. 4. The statistical results of this series of experiments are shown in detail in Fig. 5.

### II: MEASUREMENT OF THE SYSTOLIC BLOOD PRESSURE

The test was carried out as described above, however values of the systolic blood pressure were analysed.

Without addition of Triamterene/Hydrochlorothiazide to the salt/sugar cocktail systolic blood pressure remains essentially unchanged, or even slightly decreases (Fig. 6). Concomitant intake of Triamterene and Hydrochlorothiazide with the salt/sugar cocktail leads to a transient rise in systolic blood pressure with a maximum after about 40 minutes (see Fig. 7). The smaller the increase in systolic blood pressure, the better is the eGC's function with regard to sodium buffering. The higher the increase in systolic blood pressure, the weaker the eGC function.

The means of these changes in systolic blood pressure are depicted in Fig. 8. As expected, variations (deviations from the mean, see the chart of Fig. 8) are particularly substantial after 40 minutes. These changes constitute the prognostic factor. As can be taken from the individual values (Fig. 7) one subject shows hardly any response to the combination of the invention while another subject shows a marked response, depending on the functional state of the eGC.

The compositions, methods, procedures, treatments, molecules and specific compounds described herein are presently representative of preferred embodiments are exemplary and are not intended as limitations on the scope of the invention.. The listing or discussion of a previously published document in this specification should not necessarily be taken as an acknowledgement that the document is part of the state of the art or is common general knowledge.

The disclosure illustratively described herein may suitably be practiced in the absence of any element or elements, limitation or limitations, not specifically disclosed herein. Thus, for example, the terms "comprising", "including," containing", etc. shall be read expansively and without limitation.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. A combination of an epithelial sodium channel blocker and sodium chloride, wherein the combination comprises sodium chloride in an amount of 1.5 g or more.

2. The combination of claim 1, wherein the epithelial sodium channel blocker is at least one of Triamterene, Amiloride and Benzyl Amiloride.

3. The combination of claim 1 or 2, being comprised in a single pharmaceutical composition or in an aqueous medium.

4. The combination of claim 3, being comprised in an aqueous medium, wherein the concentration of sodium chloride is about 25 g/l.

5. The combination of any of claims 1-4, comprising Triamterene in an amount of about 100 mg and/or Amiloride in an amount of about 10 mg.

6. The combination of any of claims 1-5, further comprising a monosaccharide.

7. The combination of claim 6, wherein the ratio of sodium chloride to the monosaccharide is about 2 : 1.

8. The combination of claim 6 or 7, wherein the monosaccharide is glucose and wherein the combination comprises about 7.8 g glucose.

9. The combination of any of claims 1-8 for use in diagnosis and/or stratifying the risk of a disorder.

10. The combination of any of claims 1-9 for use in diagnosis of an increased risk of developing hypertension and/or stratifying the risk of developing hypertension.

11. The combination of claims 9 or 10, wherein the diagnosis and/or stratifying the risk of a disorder comprises:
(a) administration of an epithelial sodium channel blocker and sodium chloride to the subj ect,
(b) a wait for about 10 minutes or more,
(c) determination of at least one of systolic and diastolic blood pressure of the subject, and
(d) a comparison of the blood pressure to a control measurement or to a predetermined reference value,
wherein an increase in systolic and/or diastolic blood pressure compared to the control measurement indicates a risk of a disorder.

12. The combination of claim 11, comprising repeated determination of the systolic and/or diastolic blood pressure.

13. The combination of claim 12, wherein repeated determination is carried out in periodic intervals.

14. The combination of claims 12 or 13, wherein repeated determination is carried out in intervals of about 10 minutes.

15. A kit of parts comprising a first and a second container, wherein the first container comprises sodium chloride but is at least essentially void of an epithelial sodium channel blocker, and the second container comprises a combination of an epithelial sodium channel blocker and sodium chloride.
